# EUROPEAN PATENT APPLICATION

(11) **EP 4 628 018 A1**
(43) Date of publication of application: **08.10.2025**
(21) Application number: 24168555.1
(22) Date of filing: 04.04.2024
(51) Int. Cl.: A61B 5/349

(54) **METHOD OF ANALYZING RESIDUAL LOCAL ACTIVITY AFTER PULSED FIELD ABLATION OF A HUMAN HEART**

(71) Applicant: CathVision ApS, 2200 Copenhagen (DK)
(72) Inventor: FIRTH, Karl, 793 41 Insjön (SE); LEONARD, Olivier, 8181 Hoerl (CH); PAAMAND, Rune, 3050 Humlebæk (DK)
(74) Representative: Gottschald Patentanwälte Partnerschaft mbB

(57) **Abstract**

The invention relates to a method of analyzing residual local activity after pulsed field ablation of a human heart via a control system (1), wherein the control system (1) receives intracardiac electrogram data comprising at least one unipolar channel (7) comprising a post-PFA section (8) measured at a location in the human heart after an application of pulsed field ablation to the location, wherein the post-PFA section (8) comprises a beat interval (13), wherein the control system (1) delimits the beat interval (13), wherein the control system (1) performs an isolation routine on the beat interval (13) to reduce or remove the influence of at least one additional physiological signal component, wherein the control system (1) derives from the beat interval (13) at least one characteristic of a local activation (20) contained in the beat interval (13), wherein the control system (1) derives from the characteristic a residual activation indicator, wherein the control system (1) outputs the residual activation indicator to a user.

## Description

The present invention relates to a method of analyzing residual local activity after pulsed field ablation of a human heart according to claim 1 and to a control system adapted to perform the proposed method according to claim 15.

The present method is particularly concerned with atrial fibrillation and atrial flutter. Electrically, atrial fibrillation is chaotic activation of muscle cells of the atria. During atrial fibrillation, the atria only minimally contribute to the function of the heart. Atrial fibrillation, therefore, reduces the output of the heart but is not imminently dangerous. However, when becoming chronic, atrial fibrillation is correlated with increased morbidity and mortality. One treatment option for atrial fibrillation is ablation therapy. Ablation is the destruction of the cells that allow electrical wave re-entry to reduce chaotic activation of the atrial muscle cells.

Ablation can be performed by different methods of applying energy to cardiac muscle cells. Known are for example cryoablation and radiofrequency (RF) ablation. The present focus lies on pulsed field ablation (PFA), a comparatively new method of applying energy to cardiac muscle cells. Pulsed field ablation can be delivered quicker than other current ablation therapies and works by electroporation of cells in a high voltage pulsed electrical field. Electroporation leads to a breakdown of the cell membrane dependent on the strength of the electrical field. For ablation, irreversible electroporation leading to the destruction of the cardiac muscle cells, is the target. However, with lower amounts of energy and depending on parameters that cannot be fully overseen and measured, for example electrode-tissue contact, reversible electroporation may happen. Additionally, with increasing distance to the electrode or electrodes used for pulsed field ablation, some cells are always subject to reversible electroporation.

Reversible electroporation leads, during a recovery period of the affected cells, to effects that are hard to distinguish from irreversible electroporation. The recovery period spans tens of minutes to hours. The ultimate success of ablation depends on the size of the remaining lesion after recovery of the reversibly electroporated cells. Conventional methods of determining success of (atrial) ablation therapy depend on measurements of remaining conductivity between a source of atrial fibrillation, in particular the pulmonary veins, and the atrium. As reversible electroporation has similar effects than irreversible electroporation during the first minutes, conventional methods would measure the sum of reversibly and irreversibly electroporated cells and may conclude that PFA was successful, even if that is not the case due to reversible electroporation.

Irreversible electroporation can be achieved with higher certainty by increasing the strength or application time of the electrical field, however, that may lead to damage to adjacent tissue and blood cells. Additionally, higher output voltages may not be feasible due to ablation generator limitations or catheter design.

Amorós-Figueras et. al. (in Amorós-Figueras, G., Casabella-Ramon, S., Moreno-Weidmann, Z., Ivorra, A., Guerra, J. M., & Garcia-Sanchez, T. (2023). Dynamics of High-Density Unipolar Epicardial Electrograms During PFA. In Circulation: Arrhythmia and Electrophysiology (Vol. 16, Issue 9). Ovid Technologies (Wolters Kluwer Health). https://doi.org/10.1161/circep.123.011914; and in "Changes in local endocardial electrograms immediately after PFA show dose-dependent variations", EHRA 2024 abstract, G. Amoros-Figueras, Z. Zoraida Moreno-Weidmann, S Sergi Casabella-Ramon, A. Ivorra, J. Guerra, T. Garcia-Sanchez) analyzed the response of tissue of domestic swine to pulsed field ablation and observed different responses of the tissue depending on the distance to the electrode applying the pulsed field ablation, i.e. dependent on the strength of the applied electrical field. According to Amorós-Figueras, within a minute of pulsed field ablation at 1000 V, a remaining RS amplitude of about 0.5 to 0.8 millivolts can be observed even at the center of the pulsed field ablation. Whether the observed RS amplitude was caused by local activations or strong near field interference was not discussed.

Duytschaever et. al. (Duytschaever, M. F., Firth, K., Leonard, O., Paamand, R., & Knecht, S. (2023). AB-452675-3 CHARACTERISTICS OF RESIDUAL SIGNALS AFTER PULSED FIELD ABLATION OF THE PULMONARY VEINS.

In Heart Rhythm (Vol. 20, Issue 5, pp. S75-S76). Elsevier BV. https://doi.org/10.1016/j.hrthm.2023.03.362) hypothesized that the presence of a remaining local activation peak may indicate reversible electroporation. However, in 71.3 % of patients, the peak persisted after further PFA application.

The inventors of this application measured the amplitude of the remaining peaks in unipolar channels of intracardiac electrograms of human patients measured during procedures for pulsed field ablation of pulmonary vein entrances. The resulting amplitude of the peaks was significantly smaller than the measurements of Amorós-Figueras et. al., between 50 and 250 microvolts, indicating that the setup of domestic swine tissue of Amorós-Figueras et. al. cannot be transferred to human PFA applications without further analysis.

US2023/0255684 A1 describes that the energy present in several bandwidths can be used to analyze the success of pulsed field ablation. One focus lies on the bandwidth of up to 8 Hz. It is known that a strong ST elevation is present after pulsed field ablation, as also visible in Amorós-Figueras et. al.. This ST elevation leads to a high energy distribution in the frequency range between 0 Hz and 8 Hz.

Reference is also made to US2021/0162210 A1, which describes methods related to pulsed field ablation, in particular possible pulse times and other parameters for the ablation.

There is a need in the field to improve the methods for indicating success of pulsed field ablation.

The invention is based on the problem of improving the known methods to better quantify the success of pulsed field ablation.

The above-noted object is solved by the features of claim 1.

The main realization of the present invention is that a local activation is present in most or all cases after pulsed field ablation, that this local activation is however often masked by other physiological signals, in particular the ST elevation and near field interference. It is therefore not the presence of a peak indicating local activation but rather the characteristics of this local activation that can be used to quantify (or qualify) the success of pulsed field ablation. As the local activation may be masked and has a small amplitude, removing or reducing other physiological signals enables the analysis of the local activation. For the present purpose, even if the local activation is not visible, the analysis is based on the assumption that it exists and is solely masked. A further realization is that bipolar electrodes are often too far apart to pick up the local activation or oriented such that they are not sensitive to local activations and it is therefore proposed to look at the unipolar signal. Further, analyzing the correct electrogram component (small window in time) is necessary as each analysis targeting the whole signal would pick up several physiological phenomena with a higher energy and greater amplitudes than the local activation. It is therefore proposed to delimit a beat interval containing the local activation.

In detail, a method of analyzing residual local activity after pulsed field ablation of a human heart via a control system, wherein the control system receives intracardiac electrogram data comprising at least one unipolar channel comprising a post-PFA section measured at a location in the human heart after an application of pulsed field ablation to the location, wherein the post-PFA section comprises a beat interval, wherein the control system delimits the beat interval, wherein the control system performs an isolation routine on the beat interval to reduce or remove the influence of at least one additional physiological signal component, wherein the control system derives from the beat interval at least one characteristic of a local activation contained in the beat interval, wherein the control system derives from the characteristic a residual activation indicator, wherein the control system outputs the residual activation indicator to a user is proposed.

There are several ways of delimiting the beat interval, even without explicitly identifying the beat interval, however, in one embodiment according to claim 2, it is proposed that the control system identifies the beat interval and/or the local activation. This may be achieved by using a ventricular or atrial beat or a pacemaker spike as a reference. These references help with identifying the correct spot to analyze.

One reason for using pulsed field ablation is that the ablation procedure can be performed quicker than with other methods of applying energy. According to claim 3 it is therefore proposed to perform the analysis quickly after the application of the pulsed field ablation. The analyzed local activation may even be the first measurable local activation after the application of pulsed field ablation.

Claim 4 relates to the detection of a peak section in the beat interval. Identifying an interval corresponding to the local activation allows analyzing a very narrow section of the channel and therefore very specifically the local activation, whether visible or not. It is known that local activations regularly comprise a down deflection on the unipolar channel of about 6 ms. Whether a recovery of the down deflection is present and how long the recovery is depends on the specifics. The peak section may therefore have a length of around 6 ms or around 12 ms. It is however also possible to include near field potentials in the analysis and the exact location of the optimal peak section may be unknown, such that also broader peak sections are conceivable.

In one embodiment according to claim 5, in the isolation routine the control system selectively reduces or removes the influence of the additional physiological component. The term "selectively" means that the respective additional physiological component is targeted and not removed for example by a mere band pass filter or wavelet transformation aiming at the local activation. It has been found that peaks relating to near field interference have a width of approximately double the width of local activations, leaving their down deflections around 12 ms width and the width of the peak including the recovery at at least 20 ms to 24 ms. Far field interference is expected to have a width of at least 20 ms. Similarly, frequency components of the interferences are known. The ST interval elevation may have a considerably broader width but can also be identified through its frequency content, down deflection width and/or down deflection amplitude for example.

A preferred characteristic is the signal energy comprised by the local activation, either its down deflection or the whole peak (claim 6). The signal energy can be derived after detecting the local activation or by analyzing a Fourier transformation or another frequency decomposition, like wavelet transformation, at a respective frequency band for the beat interval. It is possible to transform the whole channel, but the analysis is focused on the beat interval to exclude further signal components which are not the local activation.

In claim 7 it is proposed to derive the characteristic from a template analysis, in particular a wavelet analysis. A wavelet for analyzing the local activation may be formed from known wavelets or a new wavelet taking into account the frequency and time components of the local activation explained herein. A single wavelet scaled to fit the expected peak may be sufficient. A wavelet analysis may work particularly well after the additional physiological signal components have been removed, leaving the target local activation.

In one embodiment according to claim 8 it is proposed to identify the peak or a down deflection of the local activation. This identification can be used to derive the characteristic from the down deflection and/or peak and to place the peak section based on the identification, e.g. around the detected down deflection. The peak section can then be further analyzed, which is particularly helpful if the boundaries of the local activation cannot be identified with high accuracy.

The local activation in this case has a significant amount of energy in the frequency bands around 50 Hz and 60 Hz, which are the usual power line frequencies. Conventionally, often, notch filters are used to remove power line interference. A notch filter however significantly influences the remaining local activation. This is particularly relevant for unipolar channels which are influenced more by power line interference than bipolar channels. It is therefore proposed to use an adaptive filter which has a variable attenuation in the power line frequency band (claim 9). Such a filter may for example identify a template of a power line interference, e.g. one or more cycles of a sine wave, and deduct the template in the time domain from the channel. This reduces the influence of the power line interference but leaves the local activation mostly intact. For the present purposes a notch filter is seen as having a variable attenuation as its removal fraction of energy will be mostly constant.

Claim 10 relates to embodiments in which several sections of the unipolar channel are used in the analysis. For example, the PFA section may be identified to analyze the local activations following shortly after the PFA section (claim 11). Further, a reference to which the characteristic is compared may be predefined or derived from a pre-PFA section or intermediate section (claim 12).

In one embodiment according to claim 13, it is proposed to derive the residual activation indicator from several local activations. Looking at several local activations may reduce the influence of random changes and measurement errors. Further, a change in the local activations over time can be analyzed.

Measuring the unipolar channel from an ablation electrode or an electrode on the same catheter is an efficient way of measuring the lesion close to its center. As has been explained, with increasing distances to the ablation electrode the electrical field gets weaker. Measuring the outer parts of the lesion may skew the result towards reversible electroporation (claim 14). In particular if the pulsed field ablation is applied through a single electrode, this electrode is usually bigger than conventional measurement electrodes, decreasing the amplitude of measured signals from the ablation electrode (compare Nairn, D., Hunyar, D., Sanchez, J., Doessel, O., & Loewe, A. (2020). Impact of Electrode Size on Electrogram Voltage in Healthy and Diseased Tissue. In 2020 Computing in Cardiology Conference (CinC). 2020 Computing in Cardiology Conference. Computing in Cardiology. https://doi.org/10.22489/cinc.2020.146). The proposed focus on the local activation and reduction of further physiological signal components enables the use of the ablation electrode for the proposed measurements. In particular the proposed filter helps to see sharp signals with low amplitudes.

Another teaching according to claim 15, which is of equal importance, relates to a control system adapted to perform the proposed method, wherein the control system comprises a processor, a memory, a user output device, in particular a display, and a connector for receiving the intracardiac electrogram, in particular for connecting a catheter to the control system.

All explanations given with regard to the method are fully applicable. The control system may be adapted to perform any of the described method steps.

In the following, embodiments of the invention are explained with respect to the drawing. The drawing shows in
- Fig. 1,: schematically a control system with two connected catheters and a display, the catheters being inserted into a human heart,
- Fig. 2,: a surface channel, a bipolar channel and two unipolar channels with a pre-PFA section, an intermediate section and a post-PFA section,
- Fig. 3,: three examples of post-PFA sections,
- Fig. 4,: different physiological signal components contained in a beat interval,
- Fig. 5,: a comparison of a conventional filter (a)) and a preferred filter (b)) and
- Fig. 6,: deriving a template for the filter from the channel.

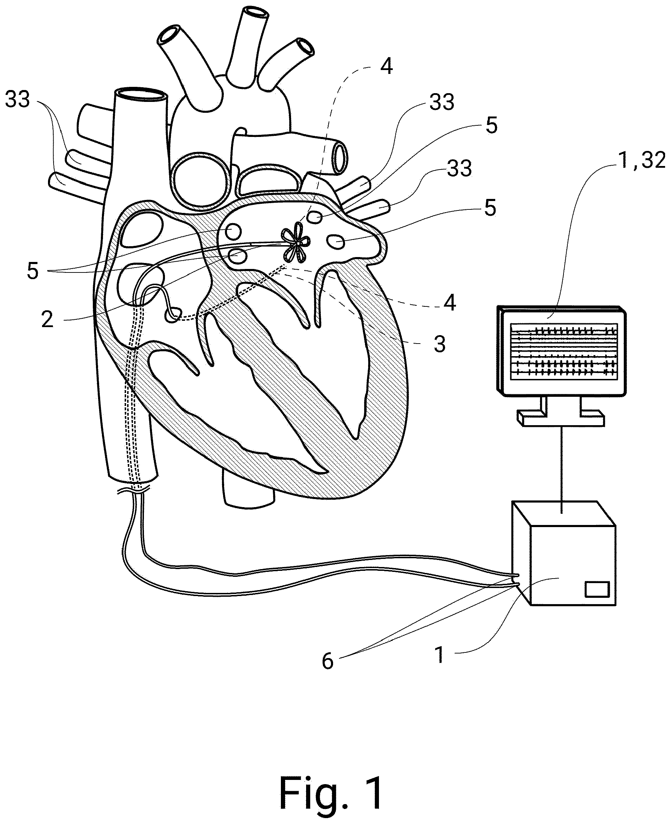

Proposed is a method of analyzing residual local activity after pulsed field ablation of a human heart via a control system 1. Fig. 1 shows schematically a human heart, an ablation catheter 2, a coronary sinus catheter 3 and a control system 1 with a display. The ablation catheter 2 and the coronary sinus catheter 3 comprise several electrodes 4 which due to their size are only indicated and not well visible. They are generally known, however.

The pulsed field ablation may be applied to an atrium of the heart, in particular the left atrium, from the inside. A preferred target for pulsed field ablation are the entrances of the pulmonary veins 5. The application of pulsed field ablation may therefore target a pulmonary vein entrance 5. An ablation therapy, which is preferably performed with the ablation catheter 2 while the control system 1 receives the intracardiac electrogram, may comprise several applications of pulsed field ablation, some or all of which may target one or more entrances of the pulmonary veins 5. Between the applications of pulsed field ablation, the ablation catheter 2 may be repositioned.

The application of pulsed field ablation may use voltages of at least 500 V, preferably at least 800 V, more preferably at least 1000 V or more than 1500 V, for example 2000 V. An application of pulsed field ablation may comprise several, for example 10, bursts, e.g. biphasic or monophasic, of a length of microseconds, preferably 10 µs to 500 µs, preferably 50 µs to 200 µs, for example 100 µs.

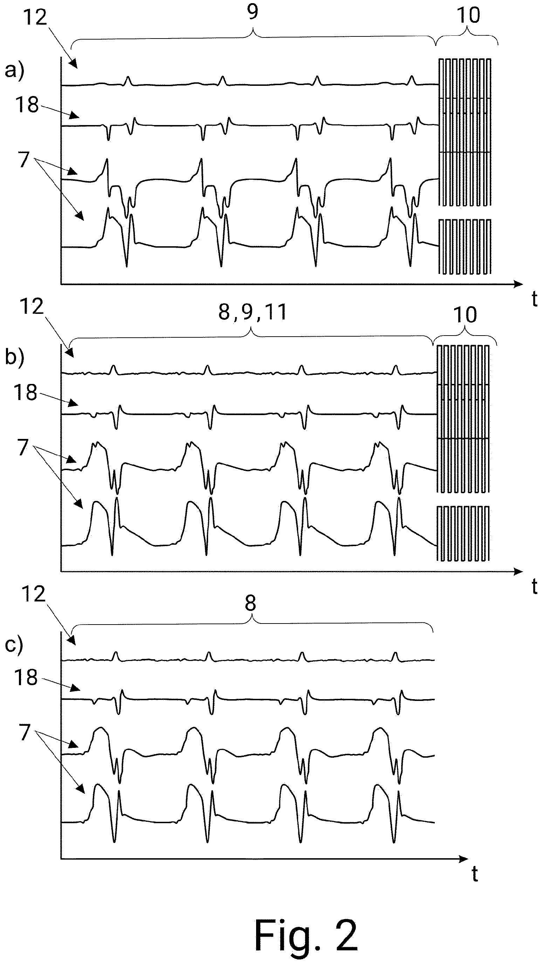

The control system 1 may be connected via a connector 6 to the ablation catheter 2 and/or the coronary sinus catheter 3 and/or a measurement catheter positioned in the, preferably right, atrium. The control system 1 receives intracardiac electrogram data comprising at least one unipolar channel 7, directly or indirectly from a catheter (Fig. 2). The control system 1 may comprise hardware for measuring potentials between electrodes 4 of the catheter and a reference electrode for the unipolar channel 7. The reference electrode may be a surface electrode for example. The hardware for measuring may comprise a hardware amplifier, a hardware filter and the like for preprocessing. Alternatively, the control system 1 may be connected to such hardware for measuring and receive the unipolar channel 7 already preprocessed.

The unipolar channel 7 comprises a post-PFA section 8 measured at a location in the human heart after an application of pulsed field ablation to the location. Fig. 2, where a), b) and c) are sections following each other in time, shows a unipolar channel 7 with a pre-PFA section 9 measured before an application of pulsed field ablation (Fig. 2 a)), two PFA sections 10 measured during applications of pulsed field ablation (Fig. 2 a) and b)), an intermediate section 11 measured between the two applications of pulsed field ablation which is further a pre-PFA section 9 and a post-PFA section 8 for one of the pulsed field ablations respectively (Fig. 2 b)) and a post-PFA section 8 (Fig. 2c)). Each mention of a pre-PFA section 9 or a post-PFA section 8 may relate to any such section or to the first pre-PFA section 9 or the last post-PFA section 8 as a preferred embodiment. The control system 1 may also receive a surface channel 12 from a surface ECG. The location is located in a radius around an ablation electrode 4 depending on the specifics of the ablation therapy. For example, the ablation catheter 2 may comprise separate measurement electrodes 4 and ablation electrodes 4.

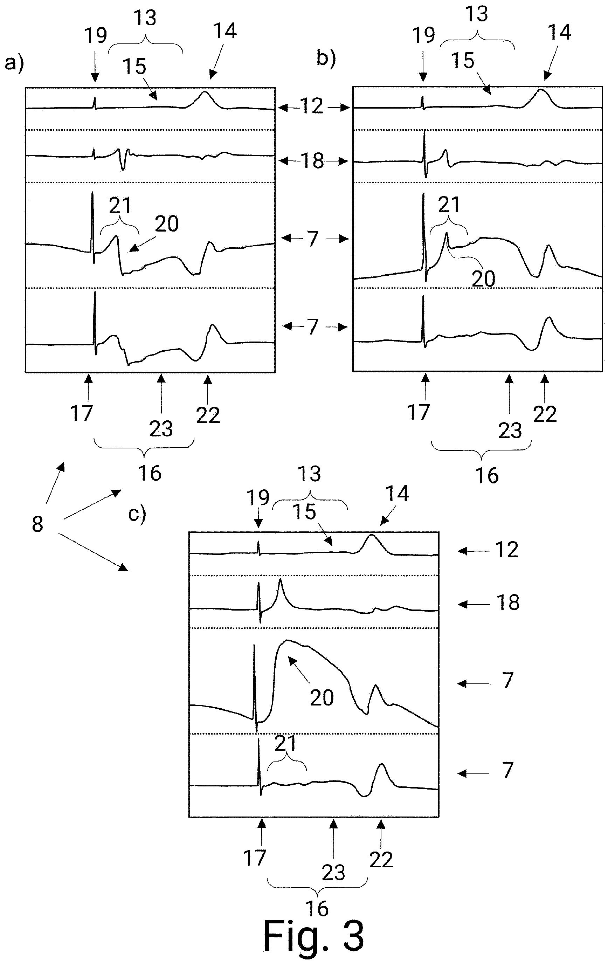

Focusing on the post-PFA section 8, the post-PFA section 8 comprises a beat interval 13. This beat interval 13 is located previous to the QRS wave 14 and comprises the time of the atrial activation leading to the P-wave 15 and the atrial ST interval 16 (also named ST segment, not to be confused with the surface ST interval which has a different timing, mostly coinciding with the surface PQ interval). If a pacemaker, implanted or temporary through a catheter electrode, is used, the beat interval 13 begins after the pacemaker time 17. Fig. 3 shows in a) to c) post-PFA sections 8 with, from top to bottom, a surface channel 12, a bipolar channel 18 and two unipolar channels 7 from the electrodes 4 forming the bipolar channel 18. The short cutouts in Fig. 3 show a pacemaker spike 19 at a pacemaker time 17, a barely visible P-wave 15 (surface channel 12) and a QRS wave 14 (surface channel 12). The intracardiac channels 7, 18 show the respective atrial signals at the same time.

In the proposed method, the control system 1 delimits the beat interval 13. To analyze the local activation 20 in the beat interval 13, it is proposed to limit the analysis to the beat interval 13 at some point during the analysis. There are many conceivable ways of delimiting the beat interval 13. The control system 1 may detect the beat interval 13 and analyze only the beat interval 13. The control system 1 may also analyze the whole unipolar channel 7, for example with a short time fourier analysis, but exclude peaks outside the beat interval 13 from further analysis. For example, knowledge about the location of the QRS wave 14 may be used to either find the beat interval 13 or exclude signals during the QRS wave 14 from further analysis, both ways leading to a delimiting of the beat interval 13. The term "delimit" is therefore to be understood broadly.

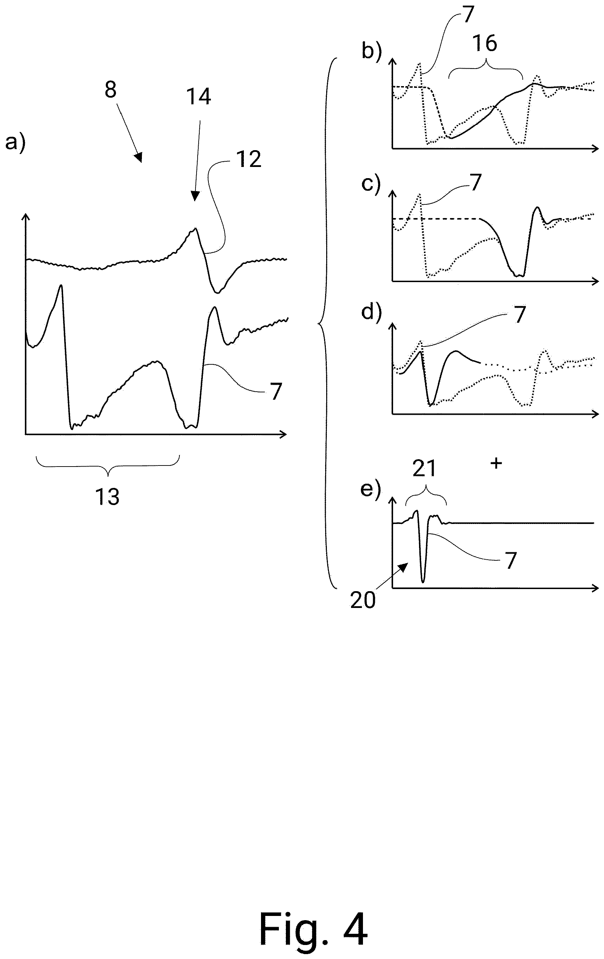

In addition to the delimiting in time, it is proposed to delimit the local activation 20 signal-contentwise. Therefore, it is proposed that the control system 1 performs an isolation routine on the beat interval 13 to reduce or remove the influence of at least one additional physiological signal component. The isolation routine may be performed selectively on the beat interval 13 or on the whole unipolar channel 7 or post-PFA section 8. Fig. 4 shows in a) a post-PFA section 8 with a surface channel 12 (top) and a unipolar channel 7 (bottom) and in b) to e) several physiological signal components, b) to d) showing additional physiological signal components and e) the remaining local activation 20 after removal of these additional physiological signal components from the unipolar channel 7. Fig. 4 b) shows the ST interval elevation, c) shows far field interference and d) shows near field interference. The influence of at least one of these, preferably at least the ST interval elevation and the far field interference, more preferably all three, is removed or reduced during the isolation routine.

The control system 1 derives from the beat interval 13 at least one characteristic of a local activation 20 contained in the beat interval 13. The characteristic is a characteristic of the local activation 20, not a global signal characteristic of an unspecific part of the unipolar channel 7. The signal characteristic may be the energy comprised in 6 ms to 20 ms around a time of the local activation 20, not however the energy comprised in 60 unspecific seconds of the unipolar channel 7.

One preferred method of removing or reducing the influence of the additional physiological signal components is focusing the analysis on a frequency band above 30 Hz to 35 Hz and preferably below 100 Hz or below 65 Hz or below 55 Hz. In the beat interval 13, in particular a yet to be explained peak section 21 comprising selectively the local activation 20, these frequency bands contain mostly energy of the local activation 20 which may serve as an indicator for a residual activation. Generally, it is proposed that the control system 1 derives from the characteristic a residual activation indicator and outputs the residual activation indicator to a user. The residual activation indicator indicates how much local activation 20 is still present near the location, in particular quantitatively. The residual activation indicator may be the characteristic itself. It is to be noted that the characteristic of the local activation 20 is more than its mere presence. On the opposite, the presence of the local activation 20 is assumed for the determination of the characteristic. Therefore, two local activations 20 which are both "present" may have different characteristics.

Preferably, the control system 1 identifies the beat interval 13 and/or the local activation 20. Preferably, the control system 1 identifies a ventricular beat time 22 corresponding to a time of an activation of the ventricles and/or an atrial beat time 23 corresponding to an activation of the atria and/or a pacemaker time 17 corresponding to a pacemaker spike 19. These times are not perfectly well-defined, e.g. the ventricular time may be the R-peak or the middle of the QRS wave 14 or another time during or near the QRS wave 14. That is acceptable for present purposes and can be taken into account when designing the algorithms for the proposed method.

Preferably, the control system 1 identifies the beat interval 13 based on the ventricular beat time 22 and/or the atrial beat time 23 and/or the pacemaker time 17, in particular as an interval with a predefined distance to the ventricular beat time 22 and/or the atrial beat time 23 and/or the pacemaker time 17 and/or as an interval with a predefined length. The control system 1 may identify several beat intervals 13 and/or local activations 20 in the post-PFA section 8 of the unipolar channel 7 and/or of several unipolar channels 7. The pacemaker spike 19 and the related pacemaker time 17 is only present if a pacemaker is used. If it is present, it is a good indicator for the timing of the local activation 20 and beat interval 13. The ventricular beat time 22 may be determined from the surface channel 12 and the atrial beat time 23 may be identified from a coronary sinus channel 24 (not shown).

It is preferably the case that the post-PFA section 8 and/or the beat interval 13 starts and/or ends within 5 minutes, preferably 3 minutes, more preferably 1 minute of an, in particular the last, application of pulsed field ablation. Analyzing the local activation 20 shortly after the application of pulsed field ablation is advantageous as the surgery should be as short as possible.

The control system 1 may derive a characteristic of at least one, preferably at least two, more preferably at least 3, local activation 20 or local activations 20 within 5 minutes, preferably 3 minutes, more preferably 1 minute of an application of pulsed field ablation.

According to one embodiment it is proposed that the control system 1 identifies a peak section 21 in the beat interval 13. The peak section 21 is a section directly related to the local activation 20. The control system 1 may derive from the peak section 21 the characteristic of the local activation 20. Herein, several possible characteristics of the local activation 20 and ways of deriving them are described. It may also be the case that several characteristics are derived and the described characteristics therefore do not need to be alternatives.

Preferably, the peak section 21 has a, in particular predefined, length of at least one of at most 50 ms, at most 40 ms, at most 30 ms, at most 20 ms, at most 15 ms, at most 12 ms, at most 10 ms, at most 8 ms, at least 4 ms, at least 5 ms, at least 6 ms. The local activation 20 comprises a down deflection of around 6 ms length. The peak section 21 may mainly contain the down deflection or may additionally contain a subsequent repolarization. Additionally, the peak section 21 may contain a margin for errors and therefore be broader than strictly necessary. The characteristic may be based on an integration of voltages of the unipolar channel 7 in the peak section 21.

The control system 1 can identify the peak section 21 based on the ventricular beat time 22 and/or the atrial beat time 23 and/or the pacemaker time 17 in particular as an interval with and/or within a predefined distance to the beat time. For example, the peak section 21 may be defined to be within 100 ms or 50 ms of a pacemaker spike 19.

According to one embodiment it is proposed that in the isolation routine the control system 1 selectively reduces or removes the influence of the additional physiological component. In the following it will be described how a wavelet analysis can be used to selectively pick out the local activation 20, thereby non-selectively reducing the influence of for example the far field interference. In particular the ST interval elevation and/or a near field interference may be removed selectively in addition thereto or independently thereof. If the ST interval elevation masks the local activation 20, removing the ST interval elevation may enhance time-domain-based analysis of the remaining signal. The additional physiological component that is selectively removed or reduced may be a far field interference and/or a near field interference and/or an ST interval elevation from the beat interval 13. The selective removal may be performed selectively at a frequency level, e.g. by a high pass filter reducing the ST interval elevation influence of mostly up to 8 Hz, and/or selectively targeting a time frame or time-domain signal component, for example via a template removal. Normal filtering as often done for intracardiac electrograms to remove components e.g. below 0.5 Hz or above 100 Hz is not aimed at physiological signal components.

In a preferred embodiment, the characteristic of the local activation 20 comprises a signal energy of the beat interval 13, in particular of the peak section 21. That may be the signal energy of the whole beat interval 13 or peak section 21 or of a part of it. Preferably, the signal energy is a signal energy at a frequency band above 10 Hz, preferably above 20 Hz, more preferably above 30 Hz, and/or, below 100 Hz, more preferably below 70 Hz, and/or, at a frequency band comprising 50 Hz and/or 60 Hz. Preferably, the characteristic does not comprise, i.e. is not relevantly influenced by, signal energy of frequency bands below 10 Hz, preferably below 20 Hz, more preferably below 30 Hz.

The control system 1 may derive the characteristic of the local activation 20 from a template analysis, in particular wavelet analysis, of the beat interval 13, in particular the peak section 21. The signal energy may be derived from a wavelet transformation transforming the unipolar channel 7 or a part of it into at least one wavelet bin via at least one wavelet. Therefore, preferably, the control system 1 derives the signal energy from the template analysis. The signal energy may be a maximum signal energy in the peak section 21 derived by transforming the peak section 21 with at least one template, in particular wavelet. The template, which may be folded over the peak section 21, may have a width of at most 50 ms, preferably at most 40 ms, more preferably at most 30 ms, more preferably at most 20 ms, more preferably at most 15 ms, more preferably at most 12 ms, more preferably at most 10 ms, more preferably at most 8 ms, and/or at least 4 ms, preferably at least 5 ms, more preferably at least 6 ms. The template analysis is preferably performed after the reduction or removal of the additional physiological signal component or components.

According to one embodiment it is proposed that the control system 1 identifies at least one down deflection of the local activation 20 and/or the local activation 20 in the beat interval 13, in particular in the peak section 21. Usual peak detection algorithms may be used. The at least one characteristic of the local activation 20 may comprise a characteristic of the down deflection, in particular an amplitude and/or slope of the down deflection.

Preferably, the control system 1 identifies the peak section 21 based on the detection of the down deflection and/or the local activation 20 as a section placed dependent on a timing of the down deflection and/or the local activation 20. In particular, the control system 1 may place the peak section 21 around the down deflection and/or the local activation 20. The control system 1 may use a known reference time, in particular the ventricular beat time 22 and/or the atrial beat time 23 and/or the pacemaker time 17, to identify the down deflection and/or local activation 20.

The characteristic of the local activation 20 may also comprise a relative timing between a reference time, in particular the ventricular beat time 22 and/or the atrial beat time 23 and/or the pacemaker time 17, and the local activation 20, which may indicate a conduction velocity around the location or whether the local activation 20 is causal for the atrial activation.

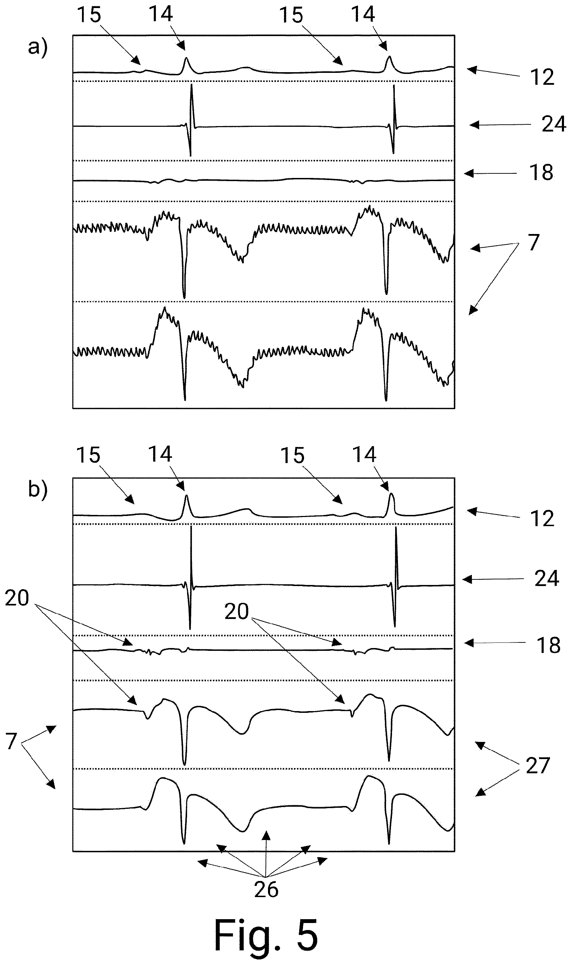

Fig. 5 shows in a) and b) from top to bottom a surface channel 12, a coronary sinus channel 24, a bipolar channel 18 and two unipolar channels 7, here without pacemaker spikes 19. Fig. 5 a) shows a conventional system where the influence of power line interference on the unipolar channels 7 is well visible. The local activation 20, corresponding in time with the P-wave 15, is barely visible in the conventional system. Fig. 5 b) shows the user of a power line filter which will now be described. In Fig. 5 b) the local activation 20 can be seen well. Conventional systems sometimes use notch filters removing basically all energy around the power line frequency. A notch filter may produce ringing and remove substantial parts of the local activations peak.

According to one embodiment it is proposed that the control system 1 applies a power line filter to the unipolar electrogram, that the power line filter has a variable attenuation, thereby removing a variable amount of energy associated with a recurrent power line noise from a power line frequency band and leaving a variable amount of energy associated with a biosignal in the power line frequency band, by detecting at least one characteristic of a power line noise and adapting the power line filter based on the detected characteristic of the power line noise. Compared to a notch filter, such an adaptive filter keeps the signal of interest.

The control system 1 preferably performs a filtering step, wherein in the filtering step the control system 1 applies a filter to the post-PFA section 8 of the unipolar channel 7, thereby generating a filtered channel section 26. The filtering step is preferably performed repeatedly, in particular continuously, preferably periodically. The control system 1 preferably receives the channel continuously, in particular in real time, and therefore applies the filter step to sections of the channel that are coming in continuously. The filtered section may be joined into a filtered channel 27 and output as such to a user and/or used by the control system 1 for further analysis.

Preferably, the filter has a variable attenuation and therefore removes a variable amount of energy associated with a recurrent noise from a frequency band and leaves a variable amount of energy associated with a biosignal in the frequency band. The biosignal is preferably the local activation 20. The biosignal may also be the ST interval elevation. The filter may leave energy associated with several biosignals. The recurrent noise is preferably a constant noise like a power line interference. Constant in that case means that the changes over time may still reach a similar amplitude as the signals that should be observed, but are significantly slower, making a good and constantly updated filter helpful.

Different from a notch filter which removes essentially all energy in a frequency band, the proposed filter leaves a substantial amount of energy in the frequency band. That enables seeing signals with a relevant amount of energy in the frequency band. Fig. 5 shows in a) that a conventional filter makes it hard to see the local activation 20 while the proposed filter in Fig. 5 b) removes the power line interference and leaves the local activation 20, which has strong signal components around 50 Hz and 60 Hz, in the channel. Fig. 5 also shows that the influence of power line interference is strong in unipolar channels 7 in conventional systems. It should be noted that the unipolar channels 7 in Fig. 5 a) have already been filtered but in a conventional manner and are not the raw signals.

It is preferably the case that the variable amount of energy removed from the unipolar channel 7 is changed over time in a filter update routine. For example, the control system 1 may estimate the energy in the frequency band stemming from noise and remove this energy. To enable analysis of the post-PFA section 8, the control system 1 performs at least one filter update routine resulting in a change of the variable amount of energy removed from the unipolar channel 7 within a time frame after the time of the application of pulsed field ablation and implements the update resulting in a filtering of the unipolar channel 7 within the time frame. The time frame is at most 60 seconds. As has been explained in the introduction, a quick signal analysis (by the system or a user) is advantageous in pulsed field ablation, on the one hand to quickly finish the surgery and on the other hand because the signals change over time. Being able to observe these very small changes demands having a good signal quality.

It may be the case, as will be explained exemplarily, that the filter update routine in the time frame differs algorithmically from the filter update routine performed previous to the application of pulsed field ablation. As the filter is preferably used in real time, the filter may be a causal filter. Preferably, the control system 1 outputs the filtered channel section 26 to the user. It may be the case that the time frame is at most 45 seconds long, preferably, that the time frame is at most 30 seconds long, more preferably, that the time frame is at most 15 seconds long. The time frame may even be at most 5 seconds or at most 1 second long.

Here and preferably, the frequency band is a power line frequency band, in particular around a power line frequency of 50 Hz or 60 Hz. Preferably, the frequency band has a width of at most 4 Hz, preferably at most 2 Hz, around the power line frequency. That takes into account that the power line frequency may change to some extent. The control system 1 may estimate the power line frequency.

It may be the case that the control system 1 identifies, in particular detects from the intracardiac electrogram, the application of the pulsed field ablation. Preferably, the control system 1 triggers and/reconfigures the filter update routine in the time frame in response to identifying the pulsed field ablation, in particular within 10 seconds of a time of the pulsed field ablation. This makes sure that the filter is quickly updated after the application of pulsed field ablation, which may have a substantial length of seconds and introduces high amounts of electrical energy.

As already mentioned, it is preferred that the biosignal is a local activation 20, and/or, that the location is inside an atrium, in particular at a pulmonary vein entrance 5.

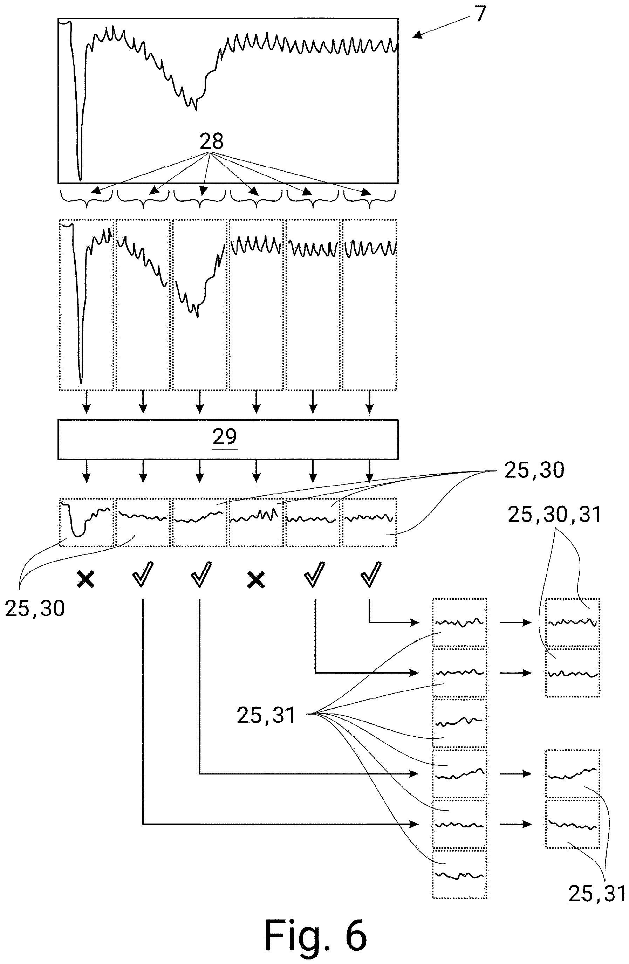

According to a preferred embodiment it is proposed that in the filter update routine the control system 1 determines an update of the filter by analyzing a current base interval 28 of the channel (Fig. 6). A current base interval 28 is a base interval 28 that is related to the timing of the filter update routine. If the filter update routine is performed repeatedly, the current base interval 28 is also changed repeatedly to a more recent base interval 28. Therefore, a sequence of filter update routines preferably uses a sequence of base intervals 28. Preferably, the base interval 28 is at most 60 seconds long, preferably at most 10 seconds, more preferably at most 1 second. The base interval 28 may have a length of at least one sample, preferably at least two samples, more preferably at least 5 ms.

According to one embodiment it is proposed that in the filter update routine in the time frame the control system 1 chooses the base interval 28 such that the base interval 28 lies within the time frame. Preferably, in the filter update routine in the time frame the base interval 28 lies between the application of pulsed field ablation and the fifth, preferably fourth, more preferably third, more preferably second, more preferably first, subsequent occurrence of the biosignal.

It may further be the case that the control system 1 performs further filter update routines after the application of pulsed field ablation, in particular in the time frame, with base intervals 28 lying between further occurrences of the biosignal, in particular with base intervals 28 lying between at least two subsequent occurrences of the biosignal.

With view towards Fig. 6 and explaining a preferred embodiment of the just mentioned, the filter may be a template filter and in the filter update routine the control system 1 may build or adapt a template 25 of the recurrent noise, in particular of at least one cycle of the recurrent noise, from the base interval 28. The control system 1 then removes, in particular subtracts, the built or adapted filter from the channel after the filter update routine. The removal may be done in the time-domain, in particular by a point by point deduction of the template 25. The template 25 may be phase-shifted for the removal. The control system 1 may use a template 25 until the conclusion of the next filter update routine and then use the new or updated template 25.

Fig. 6 shows a section of the unipolar channel 7. From this section, several base intervals 28 are chosen and their signals are analyzed to form templates 25.

The control system 1 may perform the filter update routine intermittently with unevenly spaced base intervals 28. For that, preferably, the control system 1 starts the filter update routines in regular intervals, as can be seen from the evenly spaced base intervals 28 in Fig. 6, and, based on an analysis of the signal in the base interval 28, aborts some of the filter update routines without building or adapting the template 25. In the filter update routine the control system 1 preferably analyzes a noise signal from the base interval 28, for example by using a narrow band pass filter around the frequency of the recurrent noise to remove most of the other signal components (analysis step 29 in Fig. 6). From the base interval 28, the control system 1 may derive a proposed template 30 of the noise in the base interval 28, for example of a natural number of cycles of the recurrent noise. The control system 1 may analyze the proposed template 30 derived in this manner and calculate a quality criterion, for example based on regularity of the proposed template 30. If the quality criterion does not meet a threshold, the proposed template 30 may be rejected and the filter update routine may therefore be aborted without an adaption of the template 25 used by the filter.

According to one embodiment it is proposed that the control system 1 builds a template 25 for the recurrent noise based on a base interval 28 lying prior to the application of pulsed field ablation, and, that the control system 1 adapts the template 25 based on a base interval 28 lying after the application of pulsed field ablation. Preferably, the control system 1 does not perform or always aborts the filter update routine during the application of pulsed field ablation. The control system 1 may adapt the template 25 based on the identification of pulsed field ablation. For example, the control system 1 may lower the quality criterion after the application of pulsed field ablation to improve the chances of the proposed template 30 being used for filtering the post-PFA section 8.

Fig. 6 further shows that here and preferably, the control system 1 safes several templates 25 and updates at least, in particular exactly, one of the saved templates 31 in the filter update routine according to an update criterion, in particular the quality criterion. The update criterion may be a similarity of the proposed template 30 to the saved templates 31. The updated template 25 may be a template 25 with the lowest similarity to the other saved templates 31 and/or the proposed template 30. Such an algorithm is robust against noise in the noise. However, it also hinders the update of the templates 25 after the application of pulsed field ablation if a change has occurred in the noise and that change hasn't made it into the saved templates 31.

Therefore, preferably, the control system 1 changes the update criterion in response to the identification of pulsed field ablation, thereby reconfiguring the filter update routine. For example, the control system 1 may save several templates 25 after the application of pulsed field ablation and use these templates 25 irrespective of their similarity to the previously saved templates 31. The control system 1 may also reset the filter.

The control system 1 may derive the template 25 removed from the channel from the several saved templates 31, in particular as a mean value of the saved templates 31. If exemplarily five templates 25 are saved, an update of one of the saved templates 31 may then be an update of one fifth of the used template 25. Newer templates 25 and/or templates 25 derived after the application of pulsed field ablation may be weighed higher for deriving the template 25 to be removed.

According to one embodiment it is proposed that the channel is measured in real time, and, that the control system 1 filters the channel sections in real time as they are measured and strings together the filtered channel sections 26 into a filtered channel 27. Preferably, the control system 1 displays the filtered channel 27 on a user output device 32, in particular display, to a user in real time. At least some of the channel sections may be filtered with different filters after filter update routines, as explained.

It may be the case that the control system 1 analyzes the filtered channel section 26 and/or filtered channel 27 and preferably outputs a result of the analysis, for example the residual activation indicator, to a user. The control system 1 may derive from the filtered channel section 26 and/or filtered channel 27 the residual activation indicator.

Preferably, the channel is measured with an electrode 4 of at least 6 mm², preferably at least 7 mm², more preferably at least 8 mm², surface area.

As has been explained already, the unipolar channel 7 may comprise a PFA section 10 measured while the pulsed field ablation is applied to the location and/or a pre-PFA section 9 measured before the pulsed field ablation is applied to the location. Preferably, the unipolar channel 7 comprises at least two PFA sections 10 measured during two separate applications of pulsed field ablation, and an intermediate section 11 measured between the two separate applications of pulsed field ablation, and/or a baseline section measured before the first application of pulsed field ablation to the location.

Preferably, the control system 1 detects a time of the application of pulsed field ablation, in particular from the intracardiac electrogram data. Alternatively, a trigger indicating pulsed field ablation may be received from an ablation system or the control system 1 may apply the pulsed field ablation and therefore know the time of the application. The time is preferably the end of the application of the pulsed field ablation. The control system 1 may detect the beat interval 13 and/or the local activation 20 based on the time of the application of pulsed field application, in particular as one of the following 10, preferably 8, more preferably 5, beat intervals 13 and/or local activations 20, even more preferably as the following beat interval 13 or local activation 20. Analyzing local activations 20 as soon as possible or with a defined lag towards the pulsed field ablation has the advantage that the local activations 20 are comparable between patients and the indicator is derived quickly. As Fig. 2 shows, detecting pulsed field ablation in a unipolar channel 7 or bipolar channel 18 is well possible. The control system 1 may detect several applications of pulsed field ablation and derive the characteristic and/or residual activation indicator for at least two post-PFA sections 8. This enables seeing changes through the pulsed field ablation applications. The control system 1 may detect such changes.

According to one embodiment it is proposed that the control system 1 derives the residual activation indicator by comparing the at least one characteristic of the peak with at least one reference characteristic: The reference characteristic may be predefined, or, the control system 1 may derive the reference characteristic from the pre-PFA section 9, in particular the baseline section, and/or the intermediate section 11. The reference characteristic may be based on a predefined template. The reference characteristic may be chosen as one of several reference characteristics, in particular templates, by a comparison of the baseline characteristic with the several reference characteristics. The reference characteristic may be a percentage of expected remaining energy of the local activation 20.

The control system 1 may also derive a relative timing of the local activation 20 or local activations 20 before the application of pulsed field ablation. The control system 1 may use this relative timing as a reference timing to detect the peak section 21 and/or the beat interval 13 and/or the down deflection and/or the local activation 20.

The control system 1 may derive from beat intervals 13 in the post-PFA section 8 and the intermediate section 11 and preferably the pre-PFA section 9, in particular the baseline section, at least one characteristic of a local activation 20 contained in the respective beat interval 13 for each of said beat intervals 13, and, the control system 1 may derive from said characteristics the residual activation indicator.

According to one embodiment it is proposed that the control system 1 derives from several beat intervals 13 in the post-PFA section 8 at least one characteristic of a local activation 20 contained in the respective beat interval 13 for each of said several beat intervals 13, and, that the control system 1 derives from said characteristics the residual activation indicator. Preferably, at least three, preferably at least 5, more preferably at least 7, of said several beat intervals 13 start within 5 minutes, preferably 3 minutes, more preferably 1 minute, after the application of pulsed field ablation.

The control system 1 may detect changes between local activations 20, in particular changes in a local activation morphology and/or local activation energy, and preferably derive the residual activation indicator from these changes. The characteristic may also depend on a distance in time between the local activation 20 and the pulsed field ablation. For example, energy of the local activation 20 may be valued higher if it is closer to the time of pulsed field application.

According to one embodiment it is proposed that the unipolar channel 7 is measured from an electrode 4 on a catheter, and, that the pulsed field ablation was or is applied through the same electrode 4 or another electrode 4 on the same catheter. The catheter may be connected to the control system 1. The pulsed field ablation may be delivered through one electrode 4 or through two electrodes 4 in the heart.

Additionally or alternatively, the control system 1 may detect from the characteristic of the local activation 20 in a beat interval 13 in the pre-PFA section 9, in particular from at least two unipolar channels 7, a tissue contact indicator for the electrode 4 that the unipolar channel 7 is measured with and preferably outputs the tissue contact indicator to the user. If the energy of the local activation 20 is already low previous to the pulsed field ablation, this may indicate low tissue contact of the electrode 4. If only some unipolar channels 7 show a low energy for the local activation 20, the tissue contact of these electrodes 4 may be low.

It may further be the case that the control system 1 derives from several beat intervals 13 in post-PFA sections 8 of several unipolar channels 7 measured at one or more locations after an application of pulsed field ablation to said one or more locations characteristics of local activations 20 contained in the beat intervals 13 and derives one or more residual activation indicator for said locations from a comparison of said characteristics with each other. The control system 1 may determine an indicator for a boundary of a lesion from the pulsed field ablation and/for a boundary between reversible and irreversible electroporation.

Another teaching which is of equal importance relates to a control system 1 adapted to perform the proposed method, wherein the control system 1 comprises a processor, a memory, a user output device 32, in particular a display, and a connector 6 for receiving the intracardiac electrogram, in particular for connecting a catheter to the control system 1. The control system 1 may comprise one or more microprocessors, general purpose processors, random access memories, solid state memories or other permanent memories and the like. It may comprise a user input device. The control system 1 may be adapted to perform any of the described method steps. The control system 1 may comprise instructions that when executed cause the control system 1 to perform any or all of the described method steps.

All explanations given with regard to the proposed method are fully applicable.

## Claims

1. Method of analyzing residual local activity after pulsed field ablation of a human heart via a control system (1),
wherein the control system (1) receives intracardiac electrogram data comprising at least one unipolar channel (7) comprising a post-PFA section (8) measured at a location in the human heart after an application of pulsed field ablation to the location,
wherein the post-PFA section (8) comprises a beat interval (13), wherein the control system (1) delimits the beat interval (13),
wherein the control system (1) performs an isolation routine on the beat interval (13) to reduce or remove the influence of at least one additional physiological signal component,
wherein the control system (1) derives from the beat interval (13) at least one characteristic of a local activation (20) contained in the beat interval (13),
wherein the control system (1) derives from the characteristic a residual activation indicator, wherein the control system (1) outputs the residual activation indicator to a user.

2. Method according to claim 1, **characterized in that** the control system (1) identifies the beat interval (13) and/or the local activation (20), preferably, that the control system (1) identifies a ventricular beat time (22) corresponding to a time of an activation of the ventricles and/or an atrial beat time (23) corresponding to an activation of the atria and/or a pacemaker time (17) corresponding to a pacemaker spike (19), and, that the control system (1) identifies the beat interval (13) based on the ventricular beat time (22) and/or the atrial beat time (23) and/or the pacemaker time (17), in particular as an interval with a predefined distance to the ventricular beat time (22) and/or the atrial beat time (23) and/or the pacemaker time (17) and/or as an interval with a predefined length.

3. Method according to claim 1 or 2, **characterized in that** the post-PFA section (8) and/or the beat interval (13) starts and/or ends within 5 minutes, preferably 3 minutes, more preferably 1 minute of an application of pulsed field ablation, and/or, that the control system (1) derives a characteristic of at least one, preferably at least two, more preferably at least 3, local activation (20) within 5 minutes, preferably 3 minutes, more preferably 1 minute of an application of pulsed field ablation.

4. Method according to one of the preceding claims, **characterized in that** the control system (1) identifies a peak section (21) in the beat interval (13), that the control system (1) derives from the peak section (21) the characteristic of the local activation (20), and, that the peak section (21) has a, in particular predefined, length of at least one of at most 50 ms, at most 40 ms, at most 30 ms, at most 20 ms, at most 15 ms, at most 12 ms, at most 10 ms, at most 8 ms, at least 4 ms, at least 5 ms, at least 6 ms, preferably, that the control system (1) identifies the peak section (21) based on the ventricular beat time (22) and/or the atrial beat time (23) and/or the pacemaker time (17) in particular as an interval with and/or within a predefined distance to the beat time.

5. Method according to one of the preceding claims, **characterized in that** in the isolation routine the control system (1) selectively reduces or removes the influence of the additional physiological component, preferably, that the additional physiological component is a far field interference and/or a near field interference and/or an ST interval elevation from the beat interval (13).

6. Method according to one of the preceding claims, **characterized in that** the characteristic of the local activation (20) comprises a signal energy of the beat interval (13), in particular of the peak section (21), preferably, that the signal energy is a signal energy at a frequency band above 10 Hz, preferably above 20 Hz, more preferably above 30 Hz, and/or, below 100 Hz, more preferably below 70 Hz, and/or, at a frequency band comprising 50 Hz and/or 60 Hz.

7. Method according to one of the preceding claims, **characterized in that** the control system (1) derives the characteristic of the local activation (20) from a template analysis, in particular wavelet analysis, of the beat interval (13), in particular the peak section (21), preferably, that the control system (1) derives the signal energy from the template analysis, more preferably, that the signal energy is a maximum signal energy in the peak section (21) derived by transforming the peak section (21) with at least one template.

8. Method according to one of the preceding claims, **characterized in that** the control system (1) identifies at least one down deflection of the local activation (20) and/or the local activation (20) in the beat interval (13), in particular in the peak section (21), and/or, that the at least one characteristic of the local activation (20) comprises a characteristic of the down deflection, in particular an amplitude and/or slope of the down deflection, preferably, that the control system (1) identifies the peak section (21) based on the detection of the down deflection and/or the local activation (20) as a section placed dependent on a timing of the down deflection and/or the local activation (20), in particular places the peak section (21) around the down deflection and/or the local activation (20).

9. Method according to one of the preceding claims, **characterized in that** the control system (1) applies a power line filter to the unipolar electrogram, that the power line filter has a variable attenuation, thereby removing a variable amount of energy associated with a recurrent power line noise from a power line frequency band and leaving a variable amount of energy associated with a biosignal in the power line frequency band by detecting at least one characteristic of a power line noise and adapting the power line filter based on the detected characteristic of the power line noise.

10. Method according to one of the preceding claims, **characterized in that** the unipolar channel (7) comprises a PFA section (10) measured while the pulsed field ablation is applied to the location and/or a pre-PFA section (9) measured before the pulsed field ablation is applied to the location, preferably, that the unipolar channel (7) comprises at least two PFA sections (10) measured during two separate applications of pulsed field ablation, and an intermediate section (11) measured between the two separate applications of pulsed field ablation, and/or a baseline section measured before the first application of pulsed field ablation to the location.

11. Method according to one of the preceding claims, **characterized in that** the control system (1) detects a time of the application of pulsed field ablation, in particular from the intracardiac electrogram data, that the control system (1) detects the beat interval (13) and/or the local activation (20) based on the time of the application of pulsed field application, in particular as one of the following 10, preferably 8, more preferably 5, even more preferably as the following, beat intervals (13) and/or local activations (20).

12. Method according to one of the preceding claims, **characterized in that** the control system (1) derives the residual activation indicator by comparing the at least one characteristic of the peak with at least one reference characteristic, preferably, that the reference characteristic is predefined, or, that the control system (1) derives the reference characteristic from the pre-PFA section (9) and/or the intermediate section (11).

13. Method according to one of the preceding claims, **characterized in that** the control system (1) derives from several beat intervals (13) in the post-PFA section (8) at least one characteristic of a local activation (20) contained in the respective beat interval (13) for each of said several beat intervals (13), and, that the control system (1) derives from said characteristics the residual activation indicator, preferably, that at least three, preferably at least 5, more preferably at least 7, of said several beat intervals (13) start within 5 minutes, preferably 3 minutes, more preferably 1 minute, after the application of pulsed field ablation.

14. Method according to one of the preceding claims, **characterized in that** the unipolar channel (7) is measured from an electrode (4) on a catheter, and, that the pulsed field ablation was or is applied through the same electrode (4) or another electrode (4) on the same catheter, and/or, that the control system (1) detects from the characteristic of the local activation (20) in a beat interval (13) in the pre-PFA section (9) a tissue contact indicator for the electrode (4) that the unipolar channel (7) is measured with and preferably outputs the tissue contact indicator to the user.

15. Control system adapted to perform the method according to one of the preceding claims, wherein the control system (1) comprises a processor, a memory, a user output device (32), in particular a display, and a connector (6) for receiving the intracardiac electrogram, in particular for connecting a catheter to the control system (1).
